# FASCICULE DE BREVET EUROPEEN

(11) **EP 2 986 981 B1**
(45) Date de publication et mention de la délivrance du brevet: **31.10.2018**
(21) Numéro de dépôt: 14722296.2
(22) Date de dépôt: 18.04.2014
(51) Int. Cl.: G01N 33/46, A01G 17/00, G01B 11/00

(54) **SYSTEME DE CARACTERISATION DE L'ETAT PHYSIOLOGIQUE DE VEGETAUX ET PROCEDE CORRESPONDANT**
SYSTEM ZUR CHARAKTERISIERUNG DES PHYSIOLOGISCHEN ZUSTANDS VON PFLANZEN UND ENTSPRECHENDE VERFAHREN
SYSTEM FOR CHARACTERISING THE PHYSIOLOGICAL STATE OF PLANTS AND CORRESPONDING METHOD

(30) Priorité: 18.04.2013 FR 1353542
(43) Date de publication de la demande: 24.02.2016
(73) Titulaire: E.RE.C.A. - Ingenierie en Electronique et Informat Industrielle, 69120 Vaulx en Velin (FR)
(72) Inventeur: NICOLLE, Serge, Jacques, F-69500 Bron (FR); DEBUISSON, Sébastien, F-51120 Allemant (FR)
(74) Mandataire: Weber, Jean-François
(86) Numéro de dépôt international: PCT/FR2014/050954
(87) Numéro de publication internationale: WO 2014/170620

(56) Documents cités:
- EP-A1- 0 974 262
- US-A1- 2006 272 201
- US-A1- 2007 044 445
- S. DEBUISSON ET AL: "USING MULTIPLEX AND GREENSEEKERTM TO MANAGE SPATIAL VARIATION OF VINE VIGOR IN CHAMPAGNE", PROCEEDINGS OF THE 2010 10TH INTERNATIONAL CONFERENCE ON PRECISION AGRICULTURE, 21 juillet 2010 (2010-07-21), pages 1-15, XP055082815, Denver, CO, USA

## Description

### DOMAINE TECHNIQUE

La présente invention est relative au domaine technique général du machinisme agricole, et plus précisément au secteur des outils de proxidétection permettant de collecter des données représentatives de l'état physiologique de végétaux et en particulier de végétaux cultivés en rangs, tels que des plants de vigne.

L'invention concerne plus précisément un système de caractérisation de l'état physiologique de végétaux ligneux à rameaux cultivés en rangs, du genre vignes ou arbres fruitiers, ledit système comprenant un véhicule conçu pour se déplacer entre lesdits rangs selon une direction de déplacement, et comprenant également un dispositif de géo-localisation embarqué sur ledit véhicule ainsi qu'une unité de traitement.

L'invention concerne également un procédé de caractérisation de l'état physiologique de végétaux ligneux à rameaux cultivés en rang, du genre vignes ou arbres fruitiers.

### TECHNIQUE ANTERIEURE

L'apport d'intrants (incluant en particulier des traitements phytosanitaires ou fertilisants) et la mise en oeuvre d'opérations culturales diverses, comme par exemple des opérations de taille ou de gestion de l'enherbement, sont des pratiques courantes en viticulture. Ces apports et opérations culturales sont encore à ce jour généralement mis en oeuvre de manière uniforme sur les parcelles concernées, alors même qu'il existe pourtant très souvent une variabilité spatiale au sein d'une même parcelle ou entre les parcelles, qui justifierait une mis en oeuvre différenciée, non uniforme, des apports d'intrants et des opérations culturales susvisés.

Afin de pouvoir mettre en oeuvre une telle approche différenciée, il est nécessaire de pouvoir accéder à des paramètres objectifs caractéristiques de l'éventuelle variabilité physiologique de la vigne.

A cette fin, il est connu de mettre en oeuvre des procédés de télédétection, qui reposent sur l'utilisation de capteurs de végétation embarqués sur des aéronefs (avions, drones ou satellites). Les techniques de télédétection permettent d'obtenir rapidement des mesures de paramètres sur de grandes surfaces.

Cependant, du fait du caractère discontinu de la vigne et du niveau de résolution des systèmes de télédétection connus, les informations obtenues s'avèrent généralement trop grossières pour conduire à un résultat optimal.

Il est également connu de recourir à des technologies de proxidétection reposant sur la mise en oeuvre de capteurs embarqués cette fois sur des machines viticoles terrestres. Les technologies de proxidétection connues à ce jour permettent généralement d'accéder à des informations plus précises que celles obtenues par télédétection, en contrepartie toutefois d'un temps d'acquisition généralement plus important. L'objectif de la proxidétection est ainsi de collecter automatiquement une grande série de mesures sur l'ensemble de la parcelle, afin de disposer d'une information la plus exhaustive possible quant à l'éventuelle variabilité physiologique de la vigne.

Différents outils de proxidétection sont d'ores-et-déjà disponibles, parmi lesquels un dispositif disponible sous l'appellation commerciale « *Greenseeker NDVI* ® » qui repose sur l'utilisation d'un capteur utilisant les propriétés optiques de la chlorophylle en matière d'absorption et d'émission de lumière. Le capteur en question est un capteur actif capable de générer une lumière dans le rouge et le proche infrarouge en direction de la plante, et d'une photodiode pour mesurer la lumière réfléchie. A partir de ces mesures, le capteur calcule l'indice NDVI (acronyme anglais pour « *Normalised Différence Vegetation Index»*) correspondant à un ratio entre le rouge et le proche infrarouge, variant entre -1 et 1. Ce matériel, s'il donne globalement satisfaction, n'en présente pas moins certains inconvénients. En particulier, la technologie mise en oeuvre ne permet pas de mesurer une concentration de chlorophylle : le capteur fournit en effet une valeur saturée dès les plus faibles teneurs en chlorophylle observables dans les feuilles de vignes. Il s'avère en outre que l'indice NDVI fourni par ce matériel est directement lié à la porosité du feuillage. Dans certaines situations (vignoble à faible épaisseur de végétation, tel que le vignoble champenois), la porosité est directement liée à la surface foliaire totale, généralement désignée par l'acronyme SFT). Le suivi de l'indice NDVI dans le temps et dans l'espace permet donc d'appréhender la croissance du feuillage ainsi que la variabilité de la SFT au sein de la parcelle. Cependant, dès que le feuillage s'épaissit, le capteur tend à saturer et ne permet plus d'évaluer la densité de feuillage (épaisseur). La quantité de feuillage peut être certes plus ou moins corrélée à un ensemble de paramètres agronomiques (rendement, caractéristiques des moûts...) mais il n'est pas certains que les corrélations soient forcément stables dans le temps et dans l'espace, étant entendu en outre que les pratiques culturales telles que le rognage ou le type de taille modifient la réponse du capteur. En définitive, ce matériel permet avant tout, avec les limitations exposées ci-avant, de caractériser l'expression végétative mais ne permet notamment pas d'accéder à d'autres paramètres qui pourraient s'avérer importants comme la vigueur.

On connait par ailleurs un autre matériel disponible sous l'appellation commerciale *« Multiplex Force A* ®» qui repose sur la mise en oeuvre d'un capteur optique multiparamétrique utilisant les propriétés fluorescentes de certaines familles de molécules présentes dans les feuilles et les grappes de vignes pour en estimer la teneur. Les données issues de ce matériel peuvent être plus ou moins corrélées à un ensemble de paramètres agronomiques (rendement, caractéristiques des moûts...) mais ces corrélations ne sont là encore pas forcément stables dans le temps et dans l'espace. Ce matériel permet ainsi d'estimer le métabolisme des feuilles et des grappes, mais ne permet pas là encore d'évaluer d'autres paramètres importants comme la vigueur de la vigne.

En définitive, les matériels connus brièvement décrits ci-avant permettent d'effectuer des mesures qui peuvent être reliées à des critères caractérisant l'expression végétative ou le métabolisme. Ces matériels reposent cependant avant tout sur des mesures sur feuilles et grappes. Ils ne permettent donc pas d'estimer un potentiel en début de végétation, par exemple pendant la période hivernale, avant que les feuilles ne poussent.

Afin de déterminer ce potentiel pendant la période de repos végétatif, il a été mis au point à titre expérimental un dispositif motorisé embarquant un appareil photographique numérique modifié pour prendre une photographie toutes les quatre secondes sur une parcelle de vigne. Les images ainsi obtenues sont couplées à des mesures de positions géographiques, puis traitées et analysées pour obtenir des informations relatives non seulement à l'expression végétative mais également à la vigueur de la vigne. Un tel dispositif, s'il permet d'obtenir des résultats intéressants, n'en présentent pas moins des inconvénients certains tant du point de vue de la précision des mesures que de la difficulté à traiter le volume de données recueillies.

Enfin, il existe dans l'art antérieur encore un autre dispositif permettant de mesurer la vigueur de la végétation et reposant sur une mesure de diamètre de sarment obtenue en déterminant le temps que met un sarment donné pour couper successivement deux faisceaux lumineux disposés parallèlement et à distance l'un de l'autre, et en combinant cette durée mesurée avec la vitesse d'avancement du dispositif dans le rang de végétation. Ce dispositif présente cependant lui aussi des inconvénients sérieux. En particulier, l'évaluation de la vigueur qu'il permet d'obtenir est tributaire de la connaissance de la vitesse d'avancement du dispositif, laquelle est difficilement maîtrisable avec précision et peut varier en fonction de la nature du terrain de la parcelle qui peut être plus ou moins accidentée. La nécessité de connaître avec précision la vitesse d'avancement implique non seulement de recourir à des moyens tachymétriques très fiables et précis, donc potentiellement onéreux, mais influe en outre négativement sur le volume de données à traiter (qui doivent nécessairement inclure la vitesse). Ce dispositif connu ne permet en outre pas d'établir avec précision une cartographie précise de la variabilité de l'état physiologique des végétaux de la parcelle concernée.

On connaît en outre du document US-2006/272201 un dispositif mobile permettant de mesurer le diamètre d'un sarment à l'aide d'un capteur comprenant un émetteur de faisceau lumineux et un récepteur de lumière, le diamètre en question étant déterminé à partir de la durée pendant laquelle ledit faisceau lumineux est coupé par le sarment et de la vitesse connue du véhicule.

### EXPOSE DE L'INVENTION

Les objets assignés à l'invention visent en conséquence à porter remède aux inconvénients de l'art antérieur mentionnés précédemment, et à proposer un nouveau système de caractérisation de l'état physiologique de végétaux ligneux à rameaux cultivés en rangs, qui permet d'obtenir des mesures précises, avec une excellente résolution spatiale, de façon particulièrement rapide, fiable et économique.

Un autre objet de l'invention vise à proposer un nouveau système de caractérisation de l'état physiologique de végétaux ligneux à rameaux cultivés en rangs, qui permet d'accéder de façon particulièrement simple, rapide et fiable à la biomasse bois d'une parcelle donnée.

Un autre objet de l'invention vise à proposer un nouveau système de caractérisation de l'état physiologique de végétaux ligneux à rameaux cultivés en rangs, qui permet d'obtenir rapidement une cartographie particulièrement fine et précise de la variabilité de paramètres physiologiques des végétaux ligneux cultivés sur une parcelle donnée.

Un autre objet de l'invention vise à proposer un nouveau système de caractérisation de l'état physiologique de végétaux ligneux à rameaux cultivés en rangs, qui est de construction particulièrement simple et robuste, et est adapté à toute nature de terrain.

Un autre objet de l'invention vise à proposer un nouveau système de caractérisation de l'état physiologique de végétaux ligneux à rameaux cultivés en rangs, qui est particulièrement précis.

Un autre objet de l'invention vise à proposer un nouveau système de caractérisation de l'état physiologique de végétaux ligneux à rameaux cultivés en rangs, qui repose sur la mise en oeuvre de composants standard simples, fiables et bon marché.

Un autre objet de l'invention vise à proposer un nouveau système de caractérisation de l'état physiologique de végétaux ligneux à rameaux cultivés en rangs qui puisse être utilisé par tous temps, et en particulier même par temps très ensoleillé.

Un autre objet de l'invention vise à proposer un nouveau système de caractérisation de l'état physiologique de végétaux ligneux à rameaux cultivés en rangs dont la construction est ajustable afin de s'adapter aux caractéristiques morphologiques des rangs de végétaux ligneux concernés.

Un autre objet de l'invention vise à proposer un nouveau système de caractérisation de l'état physiologique de végétaux ligneux à rameaux cultivés en rangs dont la construction repose sur la mise en oeuvre d'un minimum de composants connus et éprouvés.

Un autre objet de l'invention vise à proposer un nouveau procédé de caractérisation de l'état physiologique de végétaux ligneux à rameaux cultivés en rangs qui permet d'obtenir des mesures précises, avec une excellente résolution spatiale, de façon particulièrement rapide, fiable et économique.

Un autre objet de l'invention vise à proposer un nouveau procédé de caractérisation de l'état physiologique de végétaux ligneux à rameaux cultivés en rangs qui repose sur la mise en oeuvre de composants standards.

Un autre objet de l'invention vise à proposer un nouveau procédé de caractérisation de l'état physiologique de végétaux ligneux à rameaux cultivés en rangs qui permet d'accéder de façon extrêmement simple et rapide à une estimation de la biomasse bois d'une parcelle donnée.

Un autre objet de l'invention vise à proposer un nouveau procédé de caractérisation de l'état physiologique de végétaux ligneux à rameaux cultivés en rangs qui permet d'appréhender de façon particulièrement simple, rapide et bon marché le potentiel de développement végétatif des végétaux ligneux cultivés sur une parcelle donnée pour une année donnée.

Les objets assignés à l'invention sont atteints à l'aide d'un système de caractérisation de l'état physiologique de végétaux ligneux à rameaux cultivés en rangs, du genre vignes ou arbres fruitiers, ledit système comprenant un véhicule conçu pour se déplacer entre lesdits rangs selon une direction de déplacement, et comprenant également un dispositif de géo-localisation embarqué sur ledit véhicule ainsi qu'une unité de traitement, ledit système étant caractérisé en ce qu'il comprend un capteur incluant une source lumineuse conçue pour émettre un faisceau lumineux et un récepteur de lumière, lesdits sources et récepteurs de lumières étant montés sur ledit véhicule de façon à pouvoir être disposés de chaque côté d'un rang, afin que ledit faisceau lumineux puisse croiser, au fil de l'avancement du véhicule le long dudit rang, des rameaux appartenant aux végétaux ligneux dudit rang, ledit faisceau lumineux présentant, selon la direction de déplacement, une dimension qui est sensiblement supérieure au diamètre du plus gros des rameaux dudit rang, pour que l'interposition d'un rameau entre la source et le récepteur de lumière ne coupent que partiellement ledit faisceau lumineux et projette ainsi sur ledit récepteur de lumière une ombre à partir de laquelle l'unité de traitement détermine le diamètre local du rameau concerné, ladite unité de traitement étant en outre conçue pour associer audit diamètre local du rameau concerné des informations de géo-localisation dudit rameau en provenance du dispositif de géo-localisation.

Les objets assignés à l'invention sont également atteints à l'aide d'un procédé de caractérisation de l'état physiologique de végétaux ligneux à rameaux cultivés en rangs, du genre vignes ou arbres fruitiers, ledit procédé étant caractérisé en ce qu'il comprend les étapes suivantes :
- un capteur incluant une source lumineuse conçue pour émettre un faisceau lumineux et un récepteur de lumière est associé à un rang, de façon que lesdits sources lumineuses et récepteurs de lumières soient disposés de chaque côté dudit rang ;
- ledit capteur est déplacé le long dudit rang selon une direction de déplacement pour que ledit faisceau lumineux puisse croiser, au fil de l'avancement du capteur le long dudit rang, des rameaux appartenant aux végétaux ligneux dudit rang, ledit faisceau lumineux présentant, selon la direction de déplacement, une dimension qui est sensiblement supérieure au diamètre du plus gros des rameaux dudit rang, pour que l'interposition d'un rameau entre la source et le récepteur de lumière ne coupe que partiellement ledit faisceau lumineux et projette ainsi sur ledit récepteur de lumière une ombre à partir de laquelle le diamètre local du rameau concerné est déterminé ;
- des informations de géo-localisation dudit rameau concerné sont associées audit diamètre local.

### DESCRIPTIF SOMMAIRE DES DESSINS

D'autre particularités et avantages de l'invention apparaitront et ressortiront plus en détails à la lecture de la description faite ci-après, en référence aux dessins annexés, donnés à titre purement illustratif et non limitatif, dans lesquels :

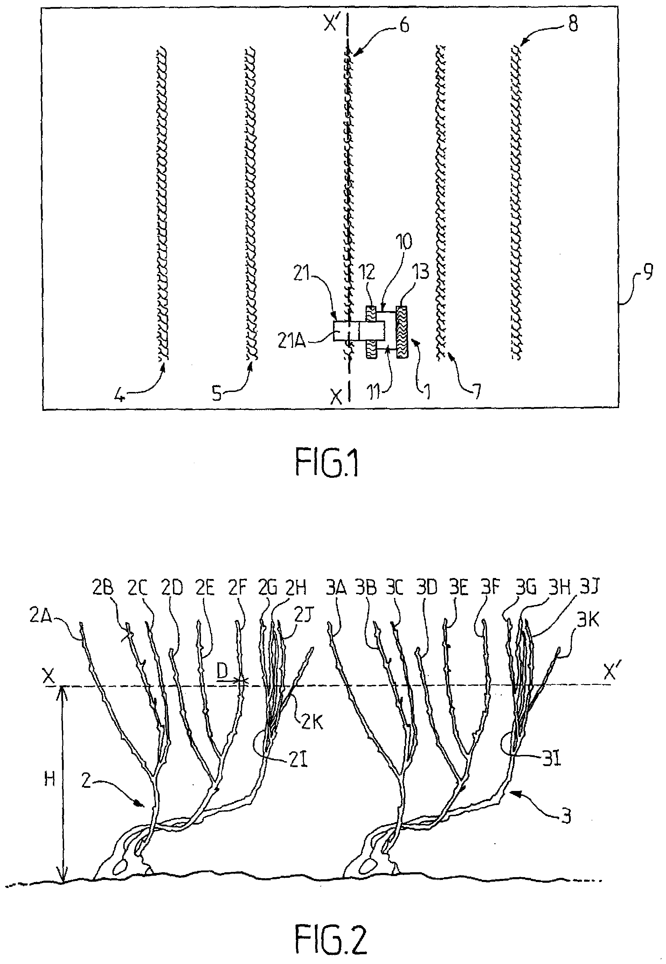

- La figure 1 illustre, selon une vue schématique de dessus, une parcelle de vigne cultivée en rangs, avec le véhicule d'un système selon l'invention en train de se déplacer entre deux rangs pour caractériser l'état physiologique des végétaux ligneux composant l'un desdits deux rangs (situé en l'espèce à gauche du véhicule du point de vue d'un observateur positionné en face de la figure 1).
- La figure 2 illustre, selon une vue schématique en élévation, deux pieds de vigne appartenant à l'un des rangs de la parcelle illustrés à la figure 1, une ligne pointillée symbolisant la direction de déplacement du véhicule du système selon l'invention, cette ligne pointillée étant placée à une altitude correspondant sensiblement à celle à laquelle sont disposés la source lumineuse et le récepteur de lumière.
- La figure 3 illustre, selon une vue schématique en perspective, un exemple de réalisation du système selon l'invention.
- La figure 4 illustre, selon une vue schématique en élévation, un détail de réalisation du système illustré à la figure 3, concernant plus précisément un tunnel embarqué sur le véhicule dudit système et au sein duquel sont disposés lesdits source lumineuse et récepteur de lumière.
- La figure 5 illustre, selon une vue schématique en coupe, le détail de réalisation de la figure 4, la source lumineuse et le récepteur de lumière étant visibles à l'intérieur dudit tunnel.
- La figure 6 illustre, selon une vue schématique de dessus, la source lumineuse et le récepteur de lumière mis en oeuvre dans le système des figures 3 à 5.

### MEILLEURE MANIERE DE REALISER L'INVENTION

L'invention concerne, selon un premier aspect, un système 1 de caractérisation de l'état physiologique de végétaux ligneux 2, 3 à rameaux 2A, 2B, 2C, 2D, 2E, 2F, 2G, 2H, 2I, 2J, 2K, 3A, 3B, 3C, 3D, 3E, 3F, 3G, 3I, 3H, 3J, 3K cultivés en rangs 4, 5, 6, 7, 8, du genre vignes ou arbres fruitiers. De préférence, comme illustré aux figures, lesdits rangs 4, 5, 6, 7, 8 sont disposés parallèlement les uns aux autres, avec un espace inter-rangs qui est avantageusement sensiblement constant sur toute la parcelle 9 concernée. Chaque rang concerné 4, 5, 6, 7, 8 est formé de plants disposés en ligne les uns derrière les autres et comprend éventuellement un système de tuteurage et/ou de palissage destiné à former une structure pour supporter les végétaux ligneux formant les rangs 4, 5, 6, 7, 8 et orienter leur croissance.

De préférence, le système 1 de caractérisation selon l'invention est spécialement adapté pour caractériser l'état physiologique de plants de vigne cultivés en rangs dans une parcelle 9 d'un vignoble. L'invention n'est cependant pas limitée à la caractérisation de l'état physiologique de plants de vigne, et peut concerner alternativement tout autre type de végétal ligneux à rameaux (arbre, arbuste ou arbrisseau fructifère ou fruitier, légumes, etc.).

Le système 1 de caractérisation selon l'invention est un système de proxidétection, et comprend un véhicule 10 conçu pour se déplacer entre lesdits rangs 4, 5, 6 7, 8 selon une direction de déplacement X-X' qui est en l'espèce sensiblement parallèle aux rangs 4, 5, 6, 7, 8 entre lesquels le véhicule 10 est amené à se déplacer. Bien entendu la dimension latérale du véhicule 10 est avantageusement conformée pour ne pas excéder la largeur de l'espace libre inter-rangs. Dans le cadre d'un vignoble, cette largeur est par exemple sensiblement inférieure ou égale à 3 mètres, et souvent inférieure ou égale à 2 mètres, voire à 1,50 mètres. Le véhicule 10 est de préférence un véhicule terrestre reposant sur le sol de la parcelle 9 et pourvu au moins d'un moteur et d'organes de liaison au sol lui permettant de se déplacer sur le sol de la parcelle 9 entre les rangs 4, 5, 6, 7, 8. Avantageusement, le véhicule 10 comprend un châssis motorisé 11 à roues, ou de façon encore plus préférentielle à chenilles 12, 13. Le recours à des chenilles 12, 13 est préféré pour permettre au véhicule 10 de se déplacer aisément y compris sur des terrains accidentés ou boueux. De préférence, le véhicule 10 comprend un chenillard, par exemple de type Niko HY22 ®, à transmission hydrostatique, propulsé par un moteur thermique (à essence) et équipé d'une batterie 12V reliée à un alternateur. L'invention n'est bien entendu pas limitée à un chenillard et d'autres types de véhicule sont envisageables (tracteur enjambeur ou toute autre machine capable de se déplacer dans une parcelle cultivée en lignes), sans pour autant que l'on sorte du cadre de l'invention.

Le système 1 selon l'invention comprend également un dispositif de géo-localisation 14 embarqué sur ledit véhicule 10 et conçu pour déterminer de façon sensiblement continue et en temps réel la position géographique instantanée du véhicule 10. Avantageusement, le dispositif de géo-localisation 14 inclut un récepteur d'un système de positionnement par satellites, comme par exemple un récepteur GPS. L'invention n'est cependant pas limitée à un moyen de géo-localisation spécifique, même si un système de positionnement par satellites est préféré. A cet égard, le recours à un récepteur GPS n'est bien entendu pas la seule possibilité envisageable dans le cadre de l'invention. Il est par exemple envisageable de mettre en oeuvre, en lieu et place d'un récepteur GPS formant un dispositif de géo-localisation 14, un récepteur Glonass (système de géo-localisation par satellites russe) ou un récepteur Galileo (système de géo-localisation par satellites européen), ou encore un récepteur GNSS (acronyme anglais de « *Global Navigation Satellites System »*) qui est un système de géo-localisation par satellites combinant les trois systèmes précités (GPS, Glonass et Galileo). Par exemple, le dispositif de géo-localisation 14 est formé par un récepteur GPS de précision centimétrique (erreur inférieure à 2,5 cm par position) de marque Trimble ® (par exemple le modèle Ag332 RTK ou Ag432 RTK radio), de préférence capable de délivrer une trame NMEA paramétrable, avec par exemple une fréquence de sortie de 1 Hz, 5 Hz, 10 Hz ou 20 Hz. Comme cela sera exposé plus en détails dans ce qui suit, la fonction du dispositif de géo-localisation 14 embarqué sur le véhicule 10 est de permettre de localiser dans l'espace l'ensemble des informations collectées par le système 1, en vue notamment d'établir des cartes de variabilités physiologiques des végétaux ligneux (formés de préférence par des plants de vigne) composant la parcelle 9 considérée.

Le système 1 de caractérisation selon l'invention comprend également une unité de traitement 15 reliée fonctionnellement au moins au dispositif de géo-localisation 14. L'unité de traitement 15 est avantageusement embarquée elle aussi sur le véhicule 10, mais il est tout à fait envisageable qu'elle ne le soit pas et soit par exemple disposée à demeure dans un bâtiment ou dans un autre véhicule. La liaison fonctionnelle entre l'unité de traitement 15 et le dispositif de géo-localisation 14 peut être une liaison filaire (comme illustrée aux figures) ou une liaison sans fil. L'unité de traitement 15 est avantageusement pourvue de moyens de traitement et de stockage de données numériques, ces moyens de traitement incluant par exemple une carte mère, un processeur, une ou plusieurs mémoires (vives et/ou mortes) et un ou plusieurs périphériques de stockage (disque dur quelle que soit sa technologie : carte mémoire, clé USB ou autre) et de commande. L'unité de traitement 15 est avantageusement pourvue de moyens de communication filaire lui permettant de se connecter à un système informatique embarqué via une liaison de transmission filaire (par exemple de type RS232, RS485, Bus I2C, Bus CAN, ProfiBus, ISOBus, UART, liaison série synchrone type SPI ou compatible, ou tout autre), ou via une liaison de transmission sans fil courte, moyenne ou grande distance (par exemple une liaison infrarouge, couplée ou non à un standard de type IrDa - liaison RFID, RF standard 433 MHz, 868 MHz, 2,4 GHz - RF couplée à un protocole standardisé type ZigBee, Bluetooth Wifi, Wimax - RF longue distance type GSM, GPRS, 3G, 4G - liaison satellitaire...). L'unité de traitement 15 peut par exemple être formée par un ordinateur, de préférence de type « *PC durci* » dans le cas où l'unité de traitement 15 est embarquée sur le véhicule 10 et donc soumise aux contraintes d'usages de ce dernier.

L'unité de traitement 15 peut être avantageusement formée par un système de traitement embarqué de type tablette ou ordiphone intégrant un système d'exploitation approprié et un logiciel permettant de mettre en oeuvre le procédé conforme à l'invention. Ce système de traitement peut avantageusement être accouplé à une ou plusieurs cartes mémoires annexes complétant les fonctions dudit système et les spécialisant pour l'application.

L'unité de traitement 15 peut par exemple être alternativement formée par un système de traitement embarqué architecturé autour d'une carte électronique de traitement embarqué du commerce, accouplée à une ou plusieurs cartes annexes complétant ses fonctions et la spécialisant pour l'application.

Selon encore une autre alternative, l'unité de traitement 15 peut par exemple être formée par un système de traitement embarqué spécifiquement conçu pour l'application et architecturé autour d'un ou plusieurs microprocesseurs, microcontrôleurs, processeurs de type DSP, circuits de type SOC (System On Chip), PSOC, SOPC, circuit de logique programmable (type FPGA, CPLD), circuit d'application spécifique (type ASIC, Gate Array). Cette carte spécifique peut être accouplée ou pas à une ou plusieurs cartes annexes complétant ses fonctions et la spécialisant pour l'application.

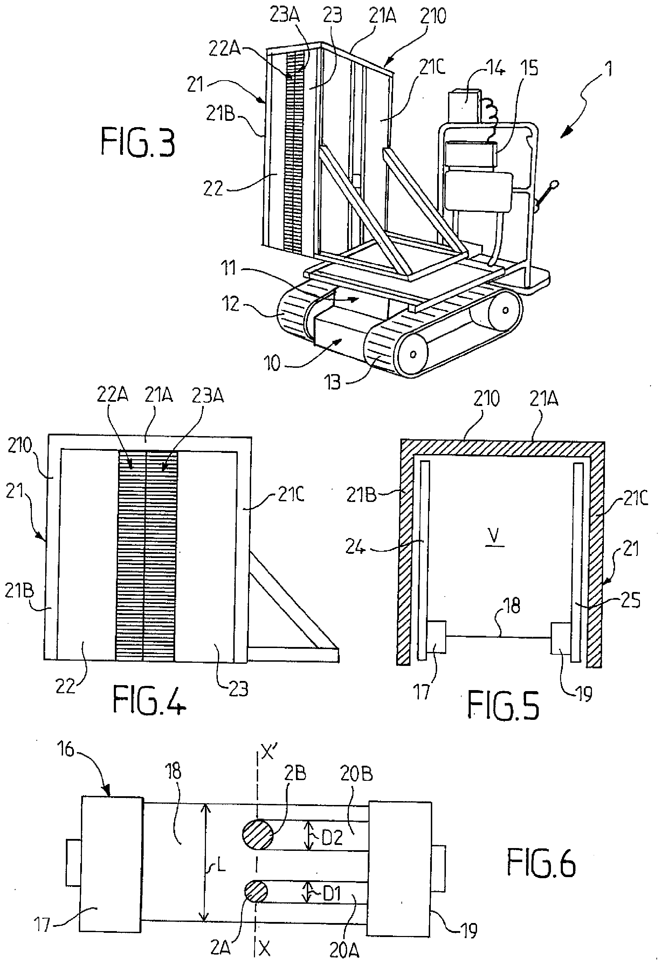

Conformément à l'invention, le système 1 comprend par ailleurs un capteur 16, représenté en tant que tel sur la figure 6, ledit capteur 16 étant de préférence entièrement embarqué sur le véhicule 10 pour collecter, au fil de l'avancement du véhicule 10 selon la direction d'avancement X-X', des informations relatives à l'état physiologique des végétaux ligneux 2, 3 composant au moins l'un des deux rangs 6, 7 entre lesquels circulent le véhicule 10. Plus précisément, le capteur 16 inclut une source lumineuse 17 conçue pour émettre un faisceau lumineux 18 et un récepteur de lumière 19 conçu et agencé pour capter, en l'absence d'obstacle entre la source lumineuse 17 et le récepteur de lumière 19, l'intégralité du faisceau lumineux 18. Le récepteur de lumière 19 est donc avantageusement monté en face de la source lumineuse 17, de façon à être frappé par le faisceau lumineux 18 émis par la source lumineuse 17.

Conformément à l'invention, les source 17 et récepteur 19 de lumière sont montés sur le véhicule 10 (c'est-à-dire embarqué sur ce dernier) de façon à pouvoir être disposés de chaque côté d'un rang 6 afin que ledit faisceau lumineux 18 puisse croiser, au fil de l'avancement du véhicule 10 le long dudit rang 6, des rameaux 2A-K, 3A-K, appartenant aux végétaux ligneux 2, 3 dudit rang 6. Les source 17 et récepteur 19 de lumière sont ainsi conçus pour se déplacer de part et d'autre d'un rang 6 donné, selon la direction de déplacement X-X', de sorte que le faisceau lumineux 18 balaye, à une altitude H, les rameaux 2A-K, 3A-K, des végétaux ligneux 2, 3 composant le rang 6 concerné. Comme cela est visible notamment sur la figure 6, le faisceau lumineux 18 présente, selon la direction de déplacement X-X', une dimension L qui est sensiblement supérieure au diamètre D du plus gros des rameaux 2A-K, 3A-K dudit rang 6 interposé entre la source lumineuse 17 et le récepteur de lumière 19, pour que l'interposition d'un rameau 2A entre la source 17 et le récepteur de lumière 19 ne coupe que partiellement ledit faisceau lumineux 18 et projette ainsi sur ledit récepteur de lumière 19 une ombre 20A à partir de laquelle l'unité de traitement 15 détermine, de préférence immédiatement (à la volée), le diamètre local D1 du rameau 2A concerné. Il est par ailleurs parfaitement envisageable, comme illustré par la figure 6, que le faisceau lumineux 18 croise sensiblement simultanément plusieurs rameaux 2A, 2B, de sorte que plusieurs ombres 20A, 20B sont projetées sur le récepteur de lumière 19, à partir desquelles l'unité de traitement 15 détermine les diamètres D1, D2 respectifs des rameaux 2A, 2B concernés, ainsi que de préférence le nombre de rameaux 2A, 2B croisant le faisceau 18.

Grâce à cette caractéristique, il est possible de déterminer directement et en temps réel le diamètre local (c'est-à-dire mesuré à l'altitude H, selon la ligne fictive matérialisant la direction de déplacement X-X') sans qu'il ne soit besoin pour cela de connaître la vitesse d'avancement du véhicule 10, laquelle peut d'ailleurs être variable en fonction de la nature du terrain de la parcelle 9 à parcourir. L'invention repose donc en particulier sur l'idée de recourir à un faisceau 18 suffisamment large pour que la dimension selon la direction de déplacement X-X' de l'ombre 20A résultant de l'éclairement du rameau 2A par le faisceau lumineux 18 soit corrélée au diamètre local D1 du rameau 2A, indépendamment de la vitesse d'avancement du véhicule 10. Afin de dériver le diamètre local D1 du rameau 2A concerné à partir des dimensions de l'ombre 20A résultant de l'éclairage, par le faisceau lumineux 18, du rameau 2A en question, le capteur 16 inclut avantageusement des moyens de calcul, comprenant par exemple un micro-processeur, un ou plusieurs programmes de calcul, etc. L'invention permet ainsi une mesure indépendante de la vitesse d'avancement du véhicule 10.

Le système 1 est avantageusement conçu pour que le faisceau lumineux 18 éclaire en permanence le récepteur de lumière 19 en n'étant que partiellement (et temporairement) occulté par les rameaux 2A-K, 3A-K croisés au fil de l'avancement du véhicule 10 le long du rang 6 concerné, ce qui permet de déterminer en temps réel et de façon séquentielle le diamètre local D1, D2 de chacun des rameaux concernés, à une altitude H donnée. Avantageusement, l'unité de traitement 15 est reliée fonctionnellement au capteur 16 afin de collecter et stocker, par tout moyen adapté (disque dur, carte mémoire, etc.) les différentes valeurs de diamètre local déterminées à l'aide du capteur 16 au fur et à mesure de l'avancement du véhicule 10 le long du rang 6 concerné. L'unité de traitement 15 est en outre conçue pour associer audit diamètre D1 du rameau concerné 2A des informations de géo-localisation dudit rameau 2A en provenance du dispositif de géo-localisation 15. En d'autres termes, l'unité de traitement 15 permet non seulement de collecter et stocker les informations de géo-localisation produites par le dispositif de géo-localisation 14 mais également d'associer ces informations de géo-localisation aux données dimensionnelles D1, D2 issues du capteur 16, afin de géo-localiser chaque mesure de diamètre local de rameau effectuée par le capteur 16, en vue de pouvoir avantageusement établir une carte de la parcelle 9 permettant d'apprécier visuellement la variabilité du diamètre des rameaux 2A-K, 3A-K à une altitude de mesure H donnée. Dans le cas où la parcelle 9 est une parcelle de vignoble composée de plants de vigne, le système 1 de caractérisation selon l'invention permet ainsi avantageusement de mesurer automatiquement et en continu des éléments caractéristiques de chaque plant de vigne, permettant d'accéder indirectement à une information sur la vigueur et l'expression de la puissance végétative de la vigne. Le système 1 permet ainsi d'une certaine façon de *« scanner »,* à l'aide du capteur 16, les rangs de vigne 4, 5, 6, 7, 8 afin de mesurer le diamètre local de chacun des rameaux 2A-K, 3A-K et d'associer à chaque diamètre local mesuré des coordonnées de géo-localisation provenant du dispositif de géo-localisation 14. Par exemple, l'unité de traitement 15 est conçue pour concaténer la trame NMEA (ou tout autre type de trame telles que les données brutes par exemple) du dispositif de géo-localisation (lorsque ce dernier est par exemple basé sur le système GPS) et les données obtenues à partir du capteur 16, pour obtenir des données concaténées qui sont de préférence ensuite enregistrées, par exemple sous la forme d'un fichier texte, sur une carte mémoire (telle qu'une carte SD, ou tout autre support de stockage comme une clé USB par exemple) pour traitement cartographique ultérieur.

Avantageusement, le système 1 de caractérisation est spécifiquement conçu pour mesurer le diamètre local D1, D2 de rameaux 2A, 2B nus c'est-à-dire dépourvu de feuilles, et de façon encore plus préférentielle de rameaux (ou bois ou sarments) nus taillés (bois de taille).

Dans le mode de réalisation préférentielle illustrée aux figures, où le système 1 est spécifiquement conçu pour caractériser l'état physiologique d'une vigne, le capteur 16 est spécifiquement dimensionné et positionné sur le véhicule 10 pour que son faisceau croise les sarments nus et de façon encore plus préférentielle les sarments nus taillés. Dans cette application préférentielle, le faisceau lumineux 18 présente, selon la direction de déplacement X-X', une dimension L qui est sensiblement supérieure ou égale à 30 millimètres, et de façon encore plus préférentielle à 26 millimètres, étant entendu qu'un sarment n'excède généralement pas 25 millimètres de diamètre. Avantageusement, l'unité de traitement 15 est conçue pour déterminer également le nombre de rameaux 2A-K, 3A-K croisés par ledit faisceau lumineux 18 au fur et à mesure de l'avancement du véhicule 10, ce qui permet par exemple de déterminer, en liaison avec les données de géo-localisation, un nombre de rameaux (et plus précisément en l'espèce de sarments) par unité de surface.

Ainsi, le système 1 selon l'invention permet d'obtenir très facilement un dénombrement sensiblement exhaustif des sarments présents dans un rang 6 de vigne, ce qui permet d'en dériver une indication sur l'expression végétative. Le système 1 selon l'invention permet également de déterminer, de préférence de façon sensiblement exhaustive, le diamètre unitaire des bois de taille (*i.e*. des sarments après la taille), ce qui donne une indication sur la vigueur de la vigne. Les éventuelles mesures issues des piquets de tuteurage/palissage et autres artefacts seront éliminées par l'unité de traitement 15 par tout traitement approprié (seuillage, etc.).

Avantageusement, la source lumineuse 17 est conçue pour émettre un faisceau laser formant ledit faisceau lumineux 18. Le recours à un faisceau laser en tant que faisceau lumineux 18 s'avère particulièrement avantageux sur le plan de la précision de mesure. Le caractère focalisé du faisceau laser prévient en effet les problèmes de précision susceptibles de survenir avec des faisceaux lumineux classiques (faisceau infrarouge par exemple) qui peuvent présenter un cône de diffraction important nuisant à la précision des mesures.

L'invention n'est cependant absolument pas limitée à la mise en oeuvre d'un faisceau lumineux de nature spécifique, et il est tout à fait envisageable de recourir à un faisceau lumineux autre qu'un faisceau laser si une précision moindre est acceptable. Avantageusement, le faisceau lumineux 18 se présente sous la forme d'un pinceau plat parallèle, comme illustré à la figure 6, ledit faisceau lumineux 18 étant avantageusement un faisceau laser en forme de pinceau plat parallèle. Avantageusement, le pinceau plat parallèle en question présente sensiblement une forme bidimensionnelle et s'étend dans un plan sensiblement parallèle à la fois à la direction de déplacement X-X' et au sol sur lequel sont plantés les rangs 4, 5, 6, 7, 8 de la parcelle 9 concernée. Le faisceau lumineux 18 présente ainsi dans ce cas sensiblement une forme de plaque rectangulaire, constituant une barrière lumineuse que chaque rameau 2A-K, 3A-K vient temporairement et partiellement occulter au fur et à mesure du déplacement du véhicule 10 le long du rang 6 concerné par les mesures.

Le pinceau plat parallèle ainsi formé est de préférence continu selon la direction de déplacement X-X', de manière à former une nappe de lumière éclairant de manière sensiblement homogène le récepteur de lumière 19 sur toute sa longueur. La continuité du pinceau plat permet d'envisager que le récepteur de lumière 19 présente une résolution suffisamment élevée, par exemple de l'ordre du dixième de millimètre, voire du micromètre ou plus, pour mesurer précisément la taille de l'ombre projetée des rameaux 2A-K, 3A-K, et en particulier la taille de l'ombre projetée des rameaux 2A-K, 3A-K, les plus petits. La nappe de lumière est préférentiellement générée par un unique émetteur, et focalisée sous la forme d'une nappe continue à l'aide d'un système optique, par exemple un jeu de miroirs et de lentilles de focalisation.

Alternativement, le pinceau plat parallèle peut être formé par une pluralité de faisceaux lasers discrets et parallèles les uns aux autres. Dans ce cas, les faisceaux lasers discrets sont préférentiellement mutuellement séparés d'une distance inférieure diamètre du rameau 2A-K, 3A-K de plus petit diamètre, voire d'une distance inférieure au dixième, ou au centième du diamètre dudit rameau de plus petit diamètre

Avantageusement, le récepteur de lumière 19 comprend un capteur CCD (dispositif à transfert de charge) qui permet, en combinaison de préférence avec un faisceau lumineux 18 formé par un faisceau laser, une excellente précision de mesure (par exemple de l'ordre du dixième de millimètre), indépendante de la quantité de lumière arrêtée par le rameau 2A, 2B concerné. Le capteur CCD permet en effet de détecter de façon très précise les bords du rameau 2A, 2B, en capturant le bord du faisceau laser arrêté par chaque rameau 2A, 2B plutôt que le volume de lumière transmis, ce qui permet d'obtenir une excellente précision de mesure.

Avantageusement, le capteur 16 est formé par un micromètre optique, et de façon encore plus préférentielle par un micromètre optique laser, par exemple de la marque KEYENCE®. En particulier, les micromètres optiques KEYENCE® de la série IG s'avèrent particulièrement adaptés aux objectifs recherchés.

Avantageusement, le système 1 selon l'invention comprend un tunnel 21 embarqué sur, ou formé par, ledit véhicule 10 et au sein duquel sont disposés lesdits source lumineuse 17 et récepteur de lumières 19, ledit tunnel 21 étant conçu pour coiffer localement le rang 6 concerné par les mesures.

Avantageusement, le tunnel 21 est pourvu à l'une de ses extrémités au moins (et de préférence à chacune de ses deux extrémités), d'un rideau d'occultation conçu pour limiter la pénétration de la lumière ambiante à l'intérieur dudit tunnel 21 tout en autorisant le déplacement du tunnel 21 le long dudit rang 6 sous l'effet du déplacement dudit véhicule 10. Le recours à un tel rideau d'occultation s'avère utile en cas de fort ensoleillement qui pourrait affecter le bon fonctionnement du capteur 16. Dans le cas où le véhicule 10 est formé par un tracteur enjambeur, les éléments du capteur 16 sont respectivement disposés sur chaque jambe de l'enjambeur ; le tunnel n'est donc plus nécessaire dans ce cas précis, puisque sa fonction est assurée par le corps même du tracteur en question, qui forme par lui-même ledit tunnel ; seul le rideau d'occultation peut avantageusement être utilisé dans ce cas si nécessaire.

Avantageusement, un support 210 pour le capteur 16 est monté sur le châssis motorisé 11. De préférence, ledit support 210 forme ledit tunnel 21. A cet effet, le support 210 affecte par exemple une forme globale de U inversé, avec une âme 21A présentant par exemple une forme de plaque sensiblement rectangulaire, à partir de laquelle s'étendent deux bras formés par exemple par deux parois latérales opaques 21B, 21C. L'âme 21A du U est destiné à venir surplomber le rang 6 objet des mesures de caractérisation par le système 1, tandis que les bras du U sont disposés de chaque côté dudit rang 6 et portent respectivement la source lumineuse 17 et le récepteur de lumière 19. Dans le cas où le véhicule 10 est formé par un tracteur enjambeur, les bras du U inversé susvisé sont avantageusement formées par des jambes dudit tracteur. Afin d'éviter que le capteur 16 ne soit soumis à la lumière ambiante (notamment lorsque celle-ci est de forte intensité), qui pourrait empêcher ou fausser les mesures, la face avant du support 21 est de préférence fermée par deux panneaux souples d'occultation 22, 23, qui forment ledit rideau d'occultation évoqué ci-avant. Lesdits panneaux souples d'occultation 22, 23 sont avantageusement opaques et conçus pour se déformer localement au passage des rameaux 2A-K, 3A-K, pour permettre le déplacement continu du support 21 le long du rang 6, tout en préservant un niveau d'obscurité suffisant dans la cavité délimitée par le support 21. Avantageusement, la face arrière du support 21 est également équipée de panneaux opaques souples (non visible aux figures) de façon à maintenir le niveau d'obscurité nécessaire au bon fonctionnement du capteur 16. Par exemple, les panneaux souples d'occultation 22, 23 peuvent chacun comprendre une bande brosse avec une zone terminale 22A, 23A formée d'une multitude de poils, de façon que lesdites brosses balayent latéralement les rameaux, autorisant ainsi un déplacement fluide et continu du support 21 tout en maintenant le niveau d'obscurité requis au niveau du capteur 16 disposé à l'intérieur du support 21, dans le volume interne V délimité par ce dernier.

Avantageusement, le capteur 16 est monté mobile à translation verticale sur le véhicule 1, afin de pouvoir régler l'altitude H à laquelle les rameaux 2A-K, 3A-K sont susceptibles de croiser le faisceau lumineux 18.

L'altitude optimale varie en effet en fonction des parcelles, selon la caractéristique propre au plan cultivé (espèce, âge, morphologie, etc.). Par exemple, la source lumineuse 17 et le récepteur de lumière 19 sont respectivement montés à coulissement vertical sur des rails verticaux 24, 25 disposés en vis-à-vis, lesdits rails verticaux 24, 25 étant par exemple respectivement solidaires des bras 21B, 21C du support 210.

Il est également envisageable qu'en plus de cette faculté de réglage de la position du capteur 16 dans le support 210, un moyen de réglage de l'altitude du support 210 dans son ensemble soit également prévu pour permettre de régler l'altitude à laquelle se positionne le support 210, en vue de s'adapter à des configurations spécifiques des rangs à étudier (présence de poteaux, de palissage, etc.).

L'invention concerne également en tant que tel un procédé de caractérisation de l'état physiologique de végétaux ligneux 2, 3 à rameaux 2A-K, 3A-K cultivés en rangs 4, 5, 6, 7, 8, du genre vignes ou arbres fruitiers, ledit procédé comprenant les étapes suivantes :
- un capteur 16, incluant une source lumineuse 17 conçue pour émettre un faisceau lumineux 18 et un récepteur de lumière 19, est associé à un rang 6, de façon que lesdits source lumineuse 17 et récepteur de lumières 19 soient disposés de chaque côté dudit rang 6 ;
- ledit capteur 16 est déplacé le long dudit rang 6 selon une direction de déplacement X-X' pour que ledit faisceau lumineux 18 puisse croiser, au fil de l'avancement du capteur 16 le long dudit rang 6, des rameaux 2A-K, 3A-K appartenant aux végétaux ligneux 2, 3 dudit rang 6, ledit faisceau lumineux 18 présentant selon la direction de déplacement X-X' une dimension L qui est sensiblement supérieure au diamètre D du plus gros des rameaux 2A-K, 3A-K dudit rang 6, pour que l'interposition d'un rameau 2A, 2B entre la source 17 et le récepteur de lumière 19 ne coupe que partiellement ledit faisceau lumineux 18 et projette ainsi sur ledit récepteur de lumière 19 une ombre 20A, 20B à partir de laquelle le diamètre local du rameau 2A, 2B concerné est déterminé ;
- des informations de géo-localisation dudit rameau concerné 2A, 2B sont associées audit diamètre local D1, D2.

Bien évidemment, le procédé selon l'invention est avantageusement mis en oeuvre à l'aide du système 1 selon l'invention précédemment décrit, de sorte que le procédé de caractérisation selon l'invention correspond de préférence à l'utilisation du système 1 décrit ci-avant. Dès lors, les éléments de description exposés dans ce qui précède en relation avec le système 1 sont également valables en ce qui concerne le procédé.

Avantageusement, le capteur 16 est déplacé sensiblement continûment le long dudit rang 6, par exemple à une vitesse moyenne comprise entre 2 et 15 km/h, et de préférence de l'ordre de 5 km/h, les opérations de détermination des diamètres des rameaux 2A-K, 3A-K s'effectuant à la volée, au fur et à mesure du déplacement. A cette fin, le capteur 16 est préférentiellement formé, comme évoqué dans ce qui précède, par un micromètre optique, et de préférence par un micromètre laser mettant en oeuvre un faisceau laser en forme de pinceau plat parallèle et un capteur CCD.

Le procédé comprend en outre avantageusement une étape de détermination du nombre de rameaux 2A-K, 3A-K croisés par le faisceau lumineux 18, afin de pouvoir accéder à une information relative à la biomasse et donc à l'expression végétative de la vigne (ou de tout autre végétal ligneux à rameaux cultivé en rangs).

Avantageusement, comme déjà évoqué dans ce qui précède, le procédé selon l'invention est mis en oeuvre alors que lesdits végétaux ligneux 2, 3 sont en période de repos végétatif et donc sensiblement dépourvus de feuilles. Par exemple, le procédé selon l'invention est avantageusement mis en oeuvre durant l'hiver, par exemple après que lesdits végétaux ligneux 2, 3 ont été taillés. De préférence, le procédé peut bien entendu être mis en oeuvre après la chute des feuilles mais avant la taille. Il s'avère que le dénombrement des bois de taille et la mesure de leurs diamètres permet d'obtenir des informations corrélées à la vigueur et à l'expression végétative des végétaux ligneux concernés (vigne ou autre). Il est ainsi possible, grâce à la géo-localisation des mesures, de cartographier la parcelle 9, ce qui s'avère extrêmement utile, notamment dans le cas d'un vignoble, pour mettre en oeuvre des traitements phytosanitaires et des opérations culturales mécaniques ou manuelles (de fertilisation, de taille...) adaptés qui tiennent compte de la variabilité intra- et inter-parcelles.

### POSSIBILITE D'APPLICATION INDUSTRIELLE

L'invention trouve son application industrielle dans la conception, la fabrication et la mise en oeuvre de systèmes de caractérisation de l'état physiologique de végétaux.

## Revendications

1. Système (1) de caractérisation de l'état physiologique de végétaux ligneux (2, 3) à rameaux (2A, 2B, 2C, 2D, 2E, 2F, 2G, 2H, 2I, 2K, 3A, 3B, 3C, 3D, 3E, 3F, 3G, 3H, 3I, 3K) cultivés en rangs (4, 5, 6, 7, 8), du genre vignes ou arbres fruitiers, ledit système (1) comprenant un véhicule (10) conçu pour se déplacer entre lesdits rangs (4, 5, 6, 7, 8) selon une direction de déplacement X-X', et comprenant également un dispositif de géo-localisation (14) embarqué sur ledit véhicule (10) ainsi qu'une unité de traitement (15), ledit système (1) étant **caractérisé en ce qu'**il comprend un capteur (16) incluant une source lumineuse (17) conçue pour émettre un faisceau lumineux (18) et un récepteur de lumière (19), lesdits source (17) et récepteur de lumière (19) étant montés sur ledit véhicule (10) de façon à pouvoir être disposés de chaque côté d'un rang (6), afin que ledit faisceau lumineux (18) puisse croiser, au fil de l'avancement du véhicule (10) le long dudit rang (6), des rameaux (2A, 2B, 2C, 2D, 2E, 2F, 2G, 2H, 2I, 2K, 3A, 3B, 3C, 3D, 3E, 3F, 3G, 3H, 3I, 3K) appartenant aux végétaux ligneux (2, 3) dudit rang (4, 5, 6, 7, 8), ledit faisceau lumineux (18) présentant, selon la direction de déplacement X-X', une dimension (L) qui est sensiblement supérieure au diamètre (D) du plus gros des rameaux dudit rang (6), pour que l'interposition d'un rameau (2A, 2B, 2C, 2D, 2E, 2F, 2G, 2H, 2I, 2K, 3A, 3B, 3C, 3D, 3E, 3F, 3G, 3H, 3I, 3K) entre la source (17) et le récepteur de lumière (19) ne coupe que partiellement ledit faisceau lumineux (18) et projette ainsi sur ledit récepteur de lumière (19) une ombre (20A, 20B) à partir de laquelle l'unité de traitement (15) détermine le diamètre local (D1, D2) du rameau (2A, 2B) concerné, ladite unité de traitement (15) étant en outre conçue pour associer audit diamètre local (D1, D2) du rameau concerné (2A, 2B) des informations de géo-localisation dudit rameau (2A, 2B) en provenance du dispositif de géo-localisation (14).

2. Système (1) selon la revendication 1 **caractérisé en ce que** ladite unité de traitement (15) est conçue pour déterminer le nombre de rameaux (2A, 2B, 2C, 2D, 2E, 2F, 2G, 2H, 2I, 2K, 3A, 3B, 3C, 3D, 3E, 3F, 3G, 3H, 3I, 3K) croisés par ledit faisceau lumineux (18).

3. Système (1) selon la revendication 1 ou 2 **caractérisé en ce que** ledit dispositif de géo-localisation (14) inclut un récepteur d'un système de positionnement par satellites, comme par exemple un récepteur GPS.

4. Système (1) selon l'une des revendications 1 à 3 **caractérisé en ce que** ledit véhicule (10) comprend un châssis motorisé (11) à roues ou à chenilles (12, 13) sur lequel est monté un support (210) affectant une forme globale de U inversé avec une âme (21A) à partir de laquelle s'étendent deux bras (21B, 21C), l'âme (21A) du U étant destinée à venir surplomber ledit rang (6), tandis que les bras (21B, 21C) du U portent respectivement la source lumineuse (17) et le récepteur de lumière (19).

5. Système (1) selon l'une des revendications 1 à 4 **caractérisé en ce que** ladite source lumineuse (17) est conçue pour émettre un faisceau laser formant ledit faisceau lumineux (18).

6. Système (1) selon l'une des revendications 1 à 5 **caractérisé en ce que** ledit faisceau lumineux (18) se présente sous la forme d'un pinceau plat parallèle.

7. Système (1) selon l'une des revendications 1 à 6 **caractérisé en ce que** ledit récepteur de lumière (19) comprend un capteur CCD.

8. Système (1) selon l'une des revendications 1 à 7 **caractérisé en ce qu'**il comprend un tunnel (21) embarqué sur, ou formé par, ledit véhicule (10) et au sein duquel sont disposés lesdits source lumineuse (17) et récepteur de lumière (19), ledit tunnel étant conçu pour coiffer localement ledit rang (6) et étant pourvu, à l'une de ses extrémité au moins, d'un rideau d'occultation conçu pour limiter la pénétration de la lumière ambiante à l'intérieur dudit tunnel (21) tout en autorisant le déplacement du tunnel (21) le long dudit rang (6) sous l'effet du déplacement dudit véhicule (10).

9. Système (1) selon l'une des revendications 1 à 8 **caractérisé en ce que** ledit capteur (16) est monté mobile à translation verticale sur ledit véhicule (1).

10. Système selon l'une des revendications 1 à 9 **caractérisé en ce que** ledit capteur (16) est formé par un micromètre optique.

11. Procédé de caractérisation de l'état physiologique de végétaux ligneux (2, 3) à rameaux (2A, 2B, 2C, 2D, 2E, 2F, 2G, 2H, 2I, 2K, 3A, 3B, 3C, 3D, 3E, 3F, 3G, 3H, 3I, 3K) cultivés en rangs (4, 5, 6, 7, 8), du genre vignes ou arbres fruitiers, ledit procédé étant **caractérisé en ce qu'**il comprend les étapes suivantes :
- un capteur (16) incluant une source lumineuse (17) conçue pour émettre un faisceau lumineux (18) et un récepteur de lumière (19) est associé à un rang (6), de façon que lesdits source lumineuse (17) et récepteur de lumière (19) soient disposés de chaque côté dudit rang (6) ;
- ledit capteur (16) est déplacé le long dudit rang (6) selon une direction de déplacement X-X' pour que ledit faisceau lumineux (18) puisse croiser, au fil de l'avancement du capteur (16) le long dudit rang (6), des rameaux (2A, 2B, 2C, 2D, 2E, 2F, 2G, 2H, 2I, 2K, 3A, 3B, 3C, 3D, 3E, 3F, 3G, 3H, 3I, 3K) appartenant aux végétaux ligneux (2, 3) dudit rang (6), ledit faisceau lumineux (18) présentant, selon la direction de déplacement X-X', une dimension L qui est sensiblement supérieure au diamètre D du plus gros des rameaux (2A, 2B, 2C, 2D, 2E, 2F, 2G, 2H, 2I, 2K, 3A, 3B, 3C, 3D, 3E, 3F, 3G, 3H, 3I, 3K) dudit rang (6), pour que l'interposition d'un rameau (2A, 2B, 2C, 2D, 2E, 2F, 2G, 2H, 2I, 2K, 3A, 3B, 3C, 3D, 3E, 3F, 3G, 3H, 3I, 3K) entre la source (17) et le récepteur de lumière (19) ne coupe que partiellement ledit faisceau lumineux (18) et projette ainsi sur ledit récepteur de lumière (19) une ombre (20A, 20B) à partir de laquelle le diamètre local D1, D2 du rameau (2A, 2B) concerné est déterminé ;
- des informations de géo-localisation dudit rameau concerné (2A, 2B) sont associées audit diamètre local D1, D2.

12. Procédé selon la revendication 11 **caractérisé en ce que** ledit capteur (16) est déplacé sensiblement continûment le long dudit rang (6).

13. Procédé selon la revendication 11 ou 12 **caractérisé en ce que** ledit capteur (16) est formé par un micromètre optique.

14. Procédé selon l'une des revendications 11 à 13 **caractérisé en ce qu'**il comprend une étape de détermination du nombre de rameaux croisés par ledit faisceau lumineux.

15. Procédé selon l'une des revendications 11 à 14 **caractérisé en ce qu'**il est mis en oeuvre alors que lesdits végétaux ligneux (2, 3) sont en période de repos végétatif et donc sensiblement dépourvus de feuilles, par exemple durant l'hiver.

## Patentansprüche

1. System (1) zur Charakterisierung des physiologischen Zustands von in Reihen (4, 5, 6, 7, 8) angebauten Holzgewächsen (2, 3) mit Zweigen (2A, 2B, 2C, 2D, 2E, 2F, 2G, 2H, 2I, 2K, 3A, 3B, 3C, 3D, 3E, 3F, 3G, 3H, 3I, 3K) in der Art von Rebstöcken oder Obstbäumen, wobei das besagte System (1) ein Fahrzeug (10), das dazu ausgelegt ist, sich zwischen den besagten Reihen (4, 5, 6, 7, 8) gemäß einer Bewegungsrichtung X-X' zu bewegen, und auch eine im besagten Fahrzeug (10) mitgeführte Einrichtung zur Ortsbestimmung (14) sowie eine Verarbeitungseinrichtung (15) umfasst, wobei das besagte System (1) **dadurch gekennzeichnet ist, dass** es einen Sensor (16) umfasst, der eine Lichtquelle (17), die dazu ausgelegt ist, einen Lichtstrahl (18) auszusenden, und einen Lichtempfänger (19) umfasst, wobei die besagte Quelle (17) und der besagte Lichtempfänger (19) auf dem besagten Fahrzeug (10) so angebracht sind, dass sie auf jeder Seite einer Reihe (6) angeordnet sein können, damit der besagte Lichtstrahl (18) im Zuge der Fortbewegung des Fahrzeugs (10) entlang der besagten Reihe (6) Zweige (2A, 2B, 2C, 2D, 2E, 2F, 2G, 2H, 2I, 2K, 3A, 3B, 3C, 3D, 3E, 3F, 3G, 3H, 3I, 3K), die zu den Holzgewächsen (2, 3) der besagten Reihe (4, 5, 6, 7, 8) gehören, kreuzen kann, wobei der besagte Lichtstrahl (18), je nach der Bewegungsrichtung X-X' eine Abmessung (L) aufweist, die im Wesentlichen größer ist als der Durchmesser (D) des dicksten Zweiges der besagten Reihe (6), damit ein Zweig (2A, 2B, 2C, 2D, 2E, 2F, 2G, 2H, 2I, 2K, 3A, 3B, 3C, 3D, 3E, 3F, 3G, 3H, 3I, 3K), der zwischen der Quelle (17) und dem Lichtempfänger (19) zu liegen kommt, den besagten Lichtstrahl (18) nur teilweise schneidet und somit auf den besagten Lichtempfänger (19) einen Schatten (20A, 20B) wirft, von dem aus die Verarbeitungseinheit (15) den lokalen Durchmesser (D1, D2) des betroffenen Zweiges (2A, 2B) ermittelt, wobei die besagte Verarbeitungseinheit (15) zudem dazu ausgelegt ist, dem besagten lokalen Durchmesser (D1, D2) des betroffenen Zweiges (2A, 2B) Ortsbestimmungsinformationen über den besagten Zweig (2A, 2B) zuzuordnen, die aus der Ortsbestimmungseinrichtung (14) stammen.

2. System (1) nach Anspruch 1, **dadurch gekennzeichnet, dass** die besagte Verarbeitungseinheit (15) dazu ausgelegt ist, die Anzahl von Zweigen (2A, 2B, 2C, 2D, 2E, 2F, 2G, 2H, 2I, 2K, 3A, 3B, 3C, 3D, 3E, 3F, 3G, 3H, 3I, 3K) zu ermitteln, die von dem besagten Lichtstrahl (18) gekreuzt werden.

3. System (1) nach Anspruch 1 oder 2, **dadurch gekennzeichnet, dass** die besagte Ortsbestimmungseinrichtung (14) einen Empfänger eines satellitengestützten Positionierungssystems wie zum Beispiel einen GPS-Empfänger umfasst.

4. System (1) nach einem der Ansprüche 1 bis 3, **dadurch gekennzeichnet, dass** das besagte Fahrzeug (10) ein motorgetriebenes Fahrgestell (11) mit Rädern oder Raupenketten (12, 13) umfasst, an dem ein Träger (210) angebracht ist, der insgesamt die Form eines umgekehrten Us mit einem Steg (21A) besitzt, aus welchem sich zwei Arme (21B, 21C) erstrecken, wobei der Steg (21A) des Us dazu bestimmt ist, die besagte Reihe (6) zu überragen, während die Arme (21B, 21C) des Us die Lichtquelle (17) bzw. den Lichtempfänger (19) tragen.

5. System (1) nach einem der Ansprüche 1 bis 4, **dadurch gekennzeichnet, dass** die Lichtquelle (17) dazu ausgelegt ist, einen Laserstrahl auszusenden, der den besagten Lichtstrahl (18) bildet.

6. System (1) nach einem der Ansprüche 1 bis 5, **dadurch gekennzeichnet, dass** der besagte Lichtstrahl (18) die Form eines parallelen Flachpinsels aufweist

7. System (1) nach einem der Ansprüche 1 bis 6, **dadurch gekennzeichnet, dass** der besagte Lichtempfänger (19) einen CCD-Sensor umfasst.

8. System (1) nach einem der Ansprüche 1 bis 7, **dadurch gekennzeichnet, dass** es einen Tunnel (21) umfasst, der auf dem Fahrzeug (10) mitgeführt oder von diesem gebildet ist und innerhalb dessen die besagte Lichtquelle (17) und der besagte Lichtempfänger (19) angeordnet sind, wobei der besagte Tunnel dazu ausgelegt ist, die besagte Reihe (6) lokal abzudecken, und mindestens an einem seiner Enden mit einem Verdunkelungsvorhang versehen ist, der dazu ausgelegt ist, das Eindringen des Umgebungslichts innerhalb des besagten Tunnels (21) zu beschränken und gleichzeitig die Fortbewegung des Tunnels (21) entlang der besagten Reihe (6) unter der Wirkung der Fortbewegung des besagten Fahrzeugs (10) zu erlauben.

9. System (1) nach einem der Ansprüche 1 bis 8, **dadurch gekennzeichnet, dass** der besagte Sensor (16) auf dem besagten Fahrzeug (1) senkrecht beweglich angebracht ist.

10. System nach einem der Ansprüche 1 bis 9, **dadurch gekennzeichnet, dass** der besagte Sensor (16) durch ein optisches Mikrometer gebildet ist.

11. Verfahren zur Charakterisierung des physiologischen Zustands von in Reihen (4, 5, 6, 7, 8) angebauten Holzgewächsen (2, 3) mit Zweigen (2A, 2B, 2C, 2D, 2E, 2F, 2G, 2H, 2I, 2K, 3A, 3B, 3C, 3D, 3E, 3F, 3G, 3H, 3I, 3K) in der Art von Rebstöcken oder Obstbäumen, wobei das besagte Verfahren **dadurch gekennzeichnet ist, dass** es folgende Schritte aufweist:
- ein Sensor (16), der eine Lichtquelle (17), die dazu ausgelegt ist, einen Lichtstrahl (18) auszusenden, und einen Lichtempfänger (19) umfasst, wird einer Reihe (6) zugeordnet, so dass die besagte Lichtquelle (17) und der besagte Lichtempfänger (19) auf jeder Seite der besagten Reihe (6) angeordnet sein können;
- der besagte Sensor (16) wird entlang der besagten Reihe (6) gemäß einer Bewegungsrichtung X-X' bewegt, damit der besagte Lichtstrahl (18) im Zuge der Fortbewegung des Sensors (16) entlang der besagten Reihe (6) Zweige (2A, 2B, 2C, 2D, 2E, 2F, 2G, 2H, 2I, 2K, 3A, 3B, 3C, 3D, 3E, 3F, 3G, 3H, 3I, 3K), die zu den Holzgewächsen (2, 3) der besagten Reihe (6) gehören, kreuzen kann, wobei der besagte Lichtstrahl (18) je nach der Bewegungsrichtung X-X' eine Abmessung L aufweist, die im Wesentlichen größer ist als der Durchmesser D des dicksten der Zweige (2A, 2B, 2C, 2D, 2E, 2F, 2G, 2H, 2I, 2K, 3A, 3B, 3C, 3D, 3E, 3F, 3G, 3H, 3I, 3K) der besagten Reihe (6), damit ein Zweig (2A, 2B, 2C, 2D, 2E, 2F, 2G, 2H, 2I, 2K, 3A, 3B, 3C, 3D, 3E, 3F, 3G, 3H, 3I, 3K), der zwischen der Quelle (17) und dem Lichtempfänger (19) zu liegen kommt, den besagten Lichtstrahl (18) nur teilweise schneidet und somit auf den Lichtempfänger (19) einen Schatten (20A, 20B) wirft, von dem aus der lokale Durchmesser D1, D2 des betroffenen Zweiges (2A, 2B) ermittelt wird;
- Ortsbestimmungsinformationen über den besagten Zweig (2A, 2B) werden dem lokalen Durchmesser D1, D2 zugeordnet.

12. Verfahren nach Anspruch 11, **dadurch gekennzeichnet, dass** der besagte Sensor (16) im Wesentlichen kontinuierlich entlang der besagten Reihe (6) bewegt wird.

13. Verfahren nach Anspruch 11 oder 12, **dadurch gekennzeichnet, dass** der besagte Sensor (16) durch ein optisches Mikrometer gebildet wird.

14. Verfahren nach einem der Ansprüche 11 bis 13, **dadurch gekennzeichnet, dass** es einen Schritt der Ermittlung der Anzahl von Zweigen aufweist, die vom besagten Lichtstrahl gekreuzt werden.

15. Verfahren nach einem der Ansprüche 11 bis 14, **dadurch gekennzeichnet, dass** es durchgeführt wird, wenn sich die besagten Holzgewächse (2, 3) in der Vegetationsruheperiode befinden und somit im Wesentlichen ohne Blätter sind, beispielsweise während des Winters.

## Claims

1. System (1) for characterising the physiological state of ligneous plants (2, 3) with branches (2A, 2B, 2C, 2D, 2E, 2F, 2G, 2H, 2I, 2K, 3A, 3B, 3C, 3D, 3E, 3F, 3G, 3H, 3I, 3K) cultivated in rows (4, 5, 6, 7, 8) of the vine or fruit tree type, said system (1) comprising a vehicle (10) designed to move between said rows (4, 5, 6, 7, 8) in a movement direction X-X', and also comprising a geolocation device (14) installed on said vehicle (10) as well as a processing unit (15), said system (1) being **characterised in that** it comprises a sensor (16) including a light source (17) designed to emit a light beam (18) and a light receiver (19), said light source (17) and receiver (19) being mounted on said vehicle (10) so as to be able to be disposed on either side of a row (6), so that said light beam (18) can, as the vehicle (10) advances along said row (6), cross branches (2A, 2B, 2C, 2D, 2E, 2F, 2G, 2H, 21, 2K, 3A, 3B, 3C, 3D, 3E, 3F, 3G, 3H, 3I, 3K) belonging to the ligneous plants (2, 3) in said row (4, 5, 6, 7, 8), said light beam (18) having, in the movement direction X-X', a dimension (L) that is substantially greater than the diameter (D) of the largest of the branches in said row (6), so that the interposition of a branch (2A, 2B, 2C, 2D, 2E, 2F, 2G, 2H, 2I, 2K, 3A, 3B, 3C, 3D, 3E, 3F, 3G, 3H, 3I, 3K) between the light source (17) and the receiver (19) only partially cuts said light beam (18) and thus projects onto said light receiver (19) a shadow (20A, 20B) from which the processing unit (15) determines the local diameter (D1, D2) of the branch (2A, 2B) concerned, said processing unit (15) being further designed so as to associate with said local diameter (D1, D2) of the branch concerned (2A, 2B) geolocation information on said branch (2A, 2B) coming from said geolocation device (14),

2. System according to claim 1, **characterised in that** said processing unit (15) is designed to determine the number of branches (2A, 2B, 2C, 2D, 2E, 2F, 2G, 2H, 2I, 2K, 3A, 3B, 3C, 3D, 3E, 3F, 3G, 3H, 31, 3K) crossed by said light beam (18).

3. System (1) according to claim 1 or claim 2, **characterised in that** said geolocation device (14) includes a receiver of a satellite positioning system, such as for example a GPS receiver.

4. System (1) according to any of claims 1 to 3, **characterised in that** said vehicle (10) comprises a motorised chassis (11) with wheels or tracks (12, 13) on which a support (210) is mounted assuming the rough shape of an inverted U with a web (21A) from which two arms (21B, 21C) extend, the web (21A) of the U being intended to overhang said row (6), while the arms (21B, 21C) of the U carry respectively the light source (17) and the light receiver (19).

5. System (1) according to any of claims 1 to 4, **characterised in that** said light source (17) is designed to emit a laser beam forming said light beam (18).

6. System (1) according to any of claims 1 to 5, **characterised in that** said light beam (18) is in the form of a parallel flat pencil.

7. System (1) according to any of claims 1 to 6, **characterised in that** said light receiver (19) comprises a CCD sensor.

8. System (1) according to any of claims 1 to 7, **characterised in that** it comprises a tunnel (21) installed on, or formed by, said vehicle (10) and in which said light source (17) and light receiver (19) are disposed, said tunnel being designed to fit locally on top of said row (6) and being provided, at one of the ends thereof at least, with an obscuring curtain designed so as to limit the penetration of ambient light inside said tunnel (21) while allowing the movement of the tunnel (21) along said row (6) under the effect of the movement of said vehicle (10).

9. System (1) according to any of claims 1 to 8, **characterised in that** said sensor (16) is mounted for vertical translational movement on said vehicle (1).

10. System according to any of claims 1 to 9, **characterised in that** said sensor (16) is formed by an optical micrometer.

11. Method for characterising the physiological state of ligneous plants (2, 3) with branches (2A, 2B, 2C, 2D, 2E, 2F, 2G, 2H, 21, 2K, 3A, 3B, 3C, 3D, 3E, 3F, 3G, 3H, 31, 3K) cultivated in rows (4, 5, 6, 7, 8), of the vine or fruit tree type, said method being **characterised in that** it comprises the following steps:
- a sensor (16) including a light source (17) designed to emit a light beam (18) and a light receiver (19) is associated with a row (6), so that said light source (17) and light receiver (19) are disposed on either side of said row (6);
- said sensor (16) is moved along said row (6) in a movement direction X-X' so that said light beam (18) can, as the sensor (16) advances along said row (6), cross the branches (2A, 2B, 2C, 2D, 2E, 2F, 2G, 2H, 21, 2K, 3A, 3B, 3C, 3D, 3E, 3F, 3G, 3H, 3I, 3K) belonging to the ligneous plants (2, 3) in said row (6), said light beam (18) having, in the movement direction X-X', a dimension L that is substantially greater than the diameter D of the largest of the branches (2A, 2B, 2C, 2D, 2E, 2F, 2G, 2H, 21, 2K, 3A, 3B, 3C, 3D, 3E, 3F, 3G, 3H, 31, 3K) in said row (6), so that the interposing of a branch (2A, 2B, 2C, 2D, 2E, 2F, 2G, 2H, 21, 2K, 3A, 3B, 3C, 3D, 3E, 3F, 3G, 3H, 31, 3K) between the source (17) and the light receiver (19) only partially cuts said light beam (18) and thus projects onto the light receiver (19) a shadow (20A, 20B) from which the local diameter (D1, D2) of the branch (2A, 2B) concerned is determined;
- geolocation information on said branch concerned (2A, 2B) is associated with said local diameter D1, D2.

12. Method according to claim 11, **characterised in that** said sensor (16) is moved substantially continuously along said row (6).

13. Method according to claim 11 or claim 12, **characterised in that** said sensor (16) is formed by an optical micrometer.

14. Method according to any of claims 11 to 13, **characterised in that** it comprises a step of determining the number of branches crossed by said light beam.

15. Method according to any of claims 11 to 14, **characterised in that** it is implemented when said ligneous plants (2, 3) are in a period of vegetative rest and therefore substantially devoid of leaves, for example during the winter.
